# EUROPEAN PATENT SPECIFICATION

(11) **EP 4 317 208 B1**
(45) Date of publication and mention of the grant of the patent: **25.02.2026**
(21) Application number: 21934216.9
(22) Date of filing: 10.05.2021
(51) Int. Cl.: C08F 8/44, C08F 112/08, C12N 11/082, C08F 212/08, C12N 9/10, C12N 11/08, C08F 212/36

(54) **AMINATION METHOD FOR POLYSTYRENE RESIN, AND METHOD FOR IMMOBILIZING ENZYME USING AMINATED RESIN**
AMINIERUNGSVERFAHREN FÜR POLYSTYROLHARZ UND VERFAHREN ZUR IMMOBILISIERUNG EINES ENZYMS MIT AMINIERTEM HARZ
PROCÉDÉ D'AMINATION D'UNE RÉSINE DE POLYSTYRÈNE ET PROCÉDÉ D'IMMOBILISATION D'UNE ENZYME À L'AIDE D'UNE RÉSINE AMINÉE

(30) Priority: 29.03.2021 CN 202110329787
(43) Date of publication of application: 07.02.2024
(73) Proprietor: Asymchem Laboratories (Tianjin) Co., Ltd, Tianjin 300457 (CN)
(72) Inventor: HONG, Hao, Morrisville North Carolina 27560 (US); XIAO, Yi, Tianjin 300457 (CN); ZHANG, Na, Tianjin 300457 (CN); LIN, Han, Tianjin 300457 (CN); PAN, Long, Tianjin 300457 (CN); VYASA, Williams, Morrisville North Carolina 27560 (US); MA, Liteng, Tianjin 300457 (CN); CUI, Yuxia, Tianjin 300457 (CN); GAO, Yanyan, Tianjin 300457 (CN)
(74) Representative: Studio Torta S.p.A.
(86) International application number: PCT/CN2021/092799
(87) International publication number: WO 2022/205565

(56) References cited:
- WO-A1-2018/187945
- CN-A- 101 776 686
- CN-A- 106 222 157
- CN-A- 112 707 977
- CN-B- 102 260 662
- JP-A- 2003 024 800

## Description

### Technical Field

The present disclosure relates to the technical field of enzyme immobilization, in particular to an amination method for a polystyrene-type resin, and a method for immobilizing an enzyme using an aminated polystyrene-type resin.

### Background

Compared with chemical catalysts, biocatalysts (enzymes) are widely used in industrial production processes due to their high activity, selectivity and substrate specificity. Biocatalysis is therefore becoming an important part in manufacturing plans for chemical drugs, intermediates, fine chemicals and final drug molecules. Although the enzyme has the high regioselectivity, high catalytic efficiency and mild reaction conditions, free enzymes often suffer from inactivation problems due to fluctuations in temperature, pH, solvent environment and other factors. As process requirements continue to expand, so do the demands on the efficiency and economy of enzyme applications. Therefore, this requires not only enhanced enzyme activity, specificity, and productivity, but also improved enzyme recyclability and reusability.

A good solution to the aforementioned problem is to utilize heterogeneous catalyst systems by enzyme immobilization. The unique catalytic performances of the enzymes are retained, while new advantages such as high stability, easy separation and recovery, superior resuability, and process simplification can be introduced as well. The performance of the immobilized enzyme mainly depends on immobilization methods and carrier materials used. According to different binding modes between the enzyme and the carrier, common methods used for enzyme immobilization may include adsorption, covalent binding, cross-linking and encapsulation and the like. Herein the adsorption method is a combination of physical adsorption, Van der Waals force and hydrophobic effect and the like between the enzyme and the carrier, and is the most commonly used method for the immobilized enzyme because of the advantages of simple and convenient operation, large adsorption capacity and low cost in industrial applications. However, due to the weak binding force between the carrier and the enzyme by the physical adsorption, there is a problem that the enzyme is fallen off from the carrier during repeated use, so that the conversion rate is decreased. Covalent binding, on the other hand, is to link the enzyme and the carrier by a covalent bond. Therefore, the carrier must have a corresponding functional group that may react with the enzyme to generate the covalent bond. Compared with the adsorption method, the linkage between the enzyme and the carrier is stronger, and the problem that the enzyme is fallen off in the use process is sovled, thus the stability and cyclicity are better.

In the early 1970s, Davankov et. al. prepared a type of a porous polymer with unique structure and excellent performance by crosslinking of linear polystyrene or re-crosslinking of loosely crosslinked polystyrene by Friedel-Crafts reaction. This type of the reactions is known as ultra-high crosslinking reaction (or Davankov ultra-high crosslinking reaction). The porous polymer prepared by such reaction is called ultra-high crosslinked adsorption resin. Ultra-high crosslinked adsorption resin is considered as the third-generation adsorption resin, in comparison of gel-type and macroporous-type adsorption resins. The ultra-high crosslinking adsorption resin typically has the structural characteristics such as large specific surface area, small average pore size, narrow pore size distribution, and good mechanical strength. At present, it shows broad application prospects in treatment of toxic organic wastewater, extraction of traditional Chinese herbs and antibiotics, gas storage and separation, as well as several other fields.

CN 106 222 157 discloses a polyamino acid modified polystyrene resin as carrier on which an enzyme has been immobilized.

CN 102 260 662 discloses polystyrene microspheres modified with polyvinyl alcohol to form a carrier for immobilizing an enzyme.

Due to the ideal reactivity of amino groups, strong electron acceptability and easy protonation, aminated polystyrene resins have seen wide applications in the fields of separation and passivation of organic drugs, biomedical polymer materials, adsorption of contaminants in sewage treatment, polymeric catalyst supports and the like. The following methods can be used for synthesizing aminated polystyrene resin:
Nitro compound reduction method: it is an important method to synthesize the aminated polystyrene resin by reducing a nitro compound to obtain an amino compound. There are many process routes, mainly including the following modes:
Catalytic hydrogenation reduction: the catalytic hydrogenation reduction method mainly uses noble metals or alloys thereof as catalysts, such as Ni, Pd, and Pt. While the reaction conditions reach a certain temperature and pressure, the nitro group is reduced to the amino group. The method has many advantages, firstly, it may reduce the manpower investment and has the strong mechanical operability; and secondly, the yield is higher, the product purity is high, the reaction steps are controllable, and it is green and pollution-free. However, it requires noble metal catalysis during the process, and requires the high temperature and pressure, the requirements for devices are higher. These undoubtedly greatly increase the cost of material synthesis.

Hydrazine hydrate reduction method: in the method, the catalysts need to be added to promote the reaction. One commonly used catalyst is the noble metals and the alloys thereof, usually including Pd/C, Pt/C, Raney Ni and the like.

Metal reduction method: the method is applied earlier, specifically by coexisting metals with acids, and also by dissolving salt electrolytes in water to achieve the reduction reaction of the nitro group. It is known by theoretical analysis that metals with electromotive forces before H, such as Li, Na, K, and Mg, may all be used as reducing agents under certain conditions. However, the drawback is that iron sludge is generated at the end of the reaction, and it may cause serious pollution to the environment.

Bromination-amination method: A p-bromine atom is firstly introduced onto phenyl ring by electrophilic substitution reaction. The obtained resin is then reacted with LiN (SiMe₃)₂ with the existence of a palladium catalyst. Finally, p-aminopolystyrene resin is afforded with post-treatment using acid or alkali.

Chloromethylated polystyrene amination method: Firstly, polystyrene microspheres are linked to a chloromethyl in the para-position of the benzene ring by a chloromethylation reaction, and are then subjected to an amination reaction with an amination reagent to prepare aminopolystyrene. However, the method requires chloromethyl methyl ether or dichloromethyl methyl ether, these chemical reagents are harmful to human bodies and have a strong cancer risk, and are therefore widely banned for use internationally. In addition, the reaction is also accompanied by side reactions such as multi-substitution and post-crosslinking, so that the chloromethyl resin structure becomes complex.

Chloroacetylated polystyrene amination method: an acyl group is introduced into the para-position of the benzene ring by an Friedel-Crafts acylation reaction, and then aminated polystyrene is prepared by the amination reaction, as follows:

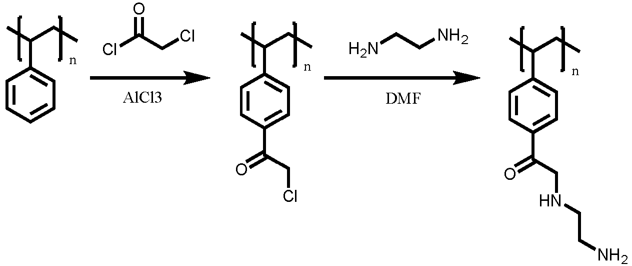

### Summary

A main purpose of the present disclosure is to provide a new amination method for a polystyrene-type resin, and to use an aminated polystyrene-type resin as an enzyme immobilization carrier to immobilize a transaminase. The method not only provides a new method for synthesis of the aminated polystyrene resin, but also provides a new class of enzyme immobilization carriers to the current library.

In order to achieve the above purpose, according to one aspect of the present disclosure, an amination method for a polystyrene-type resin is provided, and the amination method includes: in a solvent, a catalyst is used to catalyze a polystyrene-type resin and an enamine salt to perform a Friedel-Crafts alkylation reaction, to obtain an aminated polystyrene-type resin, herein the catalyst is a Lewis acid catalyst.

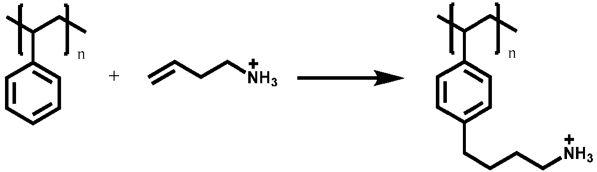

Furthermore, the temperature of the above Friedel-Crafts alkylation reaction is 40!80 °C, and preferably, the time of the Friedel-Crafts alkylation reaction is 12~20 h.

Further, the mass ratio of the above polystyrene-type resin to the enamine salt is 2:1~2:3.

Further, the molar ratio of the above enamine salt to the Lewis acid catalyst is 1:3~1:5.

Further, the above Lewis acid catalyst is selected from anhydrous aluminum trichloride or ferric trichloride.

Further, the above enamine salt is 3-butenamine hydrochloride.

Further, the above solvent is 1,2-dichloroethane.

Further, the above polystyrene-type resin is selected from any one of polystyrene resin, polystyrene-methacrylate resin, AB8 polystyrene resin, ECR1090 polystyrene resin, NKA9 polystyrene resin, D101 polystyrene resin, and SXD11 polystyrene resin.

Further, the above amination method includes: the polystyrene-type resin and enamine salt are dispersed in the solvent to form a system to be reacted; and in a nitrogen or inert atmosphere, the system to be reacted is mixed with the catalyst and heated to the temperature of the Friedel-Crafts alkylation reaction to perform the Friedel-Crafts alkylation reaction, to obtain the aminated polystyrene-type resin.

According to another aspect of the present disclosure, a method for immobilizing an enzyme using an aminated polystyrene-type resin is provided, and the method includes: the aminated polystyrene-type resin is prepared by using any one of the above amination methods; and the aminated polystyrene-type resin is used as a carrier, to immobilize the enzyme by using glutaraldehyde as a crosslinking agent.

Further, the above method includes: the aminated polystyrene-type resin is modified with a buffer solution of the glutaraldehyde, to obtain a glutaraldehyde-modified resin; and enzyme solution is crosslinked with the glutaraldehyde-modified resin, to achieve immobilization.

Further, the above method includes: the enzyme solution is mixed with the aminated polystyrene-type resin, to obtain a resin adsorbed with the enzyme; and the resin adsorbed with the enzyme is crosslinked with the buffer solution of the glutaraldehyde, to achieve immobilization.

Further, the above buffer solution of the glutaraldehyde includes a phosphate buffer solution, the mass content of the glutaraldehyde in the buffer solution of the glutaraldehyde is 1~2%, and the enzyme is a transaminase.

By applying technical schemes of the present disclosure, the present application utilizes the Friedel-Crafts alkylation reaction to graft the enamine salt onto the polystyrene-type resin in one step, and the amination of the polystyrene-type resin is completed. The conditions of the Friedel-Crafts alkylation reaction are easy to control, the post-treatment process is simple, and it is only necessary to remove the catalyst and the unreacted enamine salt by washing, such that the above amination method in the present application has few steps and is simple and easy to implement. Meanwhile, the amination method further avoids the use of a noble metal catalyst, thereby reducing the production cost. The commercial polystyrene resin modified by amination may be successfully used as an enzyme immobilization carrier, and has better immobilization effects and reusability. The present application also provides new insights for the design of enzyme immobilization carrier, so that more selections for immobilization of the different enzymes are provided in terms of carrier pore size, specific surface area, skeleton structure, carrier polarity, and other aspects.

### Detailed Description of the Embodiments

It should be noted that examples and features in the present application may be combined with each other if no other conflicts exist. The present disclosure is described in detail below in combination with examples.

As analyzed in the background of the present application, the preparation method for the aminated polystyrene-type resin in prior art may use a complex preparation method, require a plurality of steps or multiple post-treatment methods. In order to address the problem, the present application provides a facile amination method for a polystyrene-type resin, and a method for immobilizing an enzyme using an aminated polystyrene-type resin.

In a typical embodiment of the present application, an amination method for a polystyrene-type resin is provided, and the amination method includes: in a solvent, a catalyst is used to catalyze a polystyrene-type resin and an enamine salt to perform a Friedel-Crafts alkylation reaction, to obtain an aminated polystyrene-type resin, herein the catalyst is a Lewis acid catalyst.

The present application achieves amination of polystyrene-type resin by utilizing the Friedel-Crafts alkylation reaction to graft the enamine salt onto the polystyrene-type resin. The conditions of the Friedel-Crafts alkylation reaction are easy to control, the post-treatment process is simple, and it is only necessary to remove the catalyst and the unreacted enamine salt by washing. Therefore, the above amination method in the present application has fewer steps and is simple and easy to implement. Meanwhile, the amination method further avoids the use of a noble metal catalyst, thereby the production cost is reduced. The commercial polystyrene resins modified by aforementioned amination method may be successfully used as an enzyme immobilization carriers, and exhibit good immobilization effects and reusability. The present application also provides new insights for the design of enzyme immobilization carrier, so that more selections for immobilization of the different enzymes are provided in terms of carrier pore size, specific surface area, skeleton structure, carrier polarity, and other aspects.

In order to improve the efficiency of the above Friedel-Crafts alkylation reaction, for the above reaction raw materials, preferably the temperature of the above Friedel-Crafts alkylation reaction is 40~80 °C, and preferably the time of the Friedel-Crafts alkylation reaction is 12~20 h.

The above Friedel-Crafts alkylation reaction essentially involves grafting an alkyl group with an amino group onto a benzene ring of polystyrene. Due to the macromolecular characteristics of the polystyrene-type resin, it is difficult to achieve uniform grafting on each benzene ring. In order to improve the amount of amino groups grafted onto polystyrene-type resins, preferably the molar ratio of the above polystyrene-type resin to the enamine salt is 2:1~2:3.

In addition, in order to improve catalytic efficiency, avoid catalyst waste, and facilitate catalyst removal, preferably the molar ratio of the above enamine salt to the Lewis acid catalyst is 1:3~1:5.

In the above method, the Lewis acid catalyst used for the Friedel-Crafts alkylation reaction may be selected from Lewis acid catalysts commonly used for the Friedel-Crafts alkylation reaction in prior art. In order to facilitate catalyst removal and ensure efficient catalysis, preferably the above Lewis acid catalyst is any one of anhydrous aluminum trichloride and ferric trichloride or a combination thereof.

In some embodiments, the enamine salt used for the above amination method is 3-butenamine hydrochloride, to achieve efficient amination.

The above solvent used in the present application is mainly for dissolving the enamine salt and Lewis acid catalyst, in order to facilitate its full contact with the polystyrene-type resin. In order to improve the solubility of the enamine salt and Lewis acid catalyst, preferably the above solvent is 1,2-dichloroethane.

The polystyrene-type resin used in the present application includes but not limited to any one of polystyrene resin, polystyrene methacrylate resin, AB8 polystyrene resin, ECR1090 polystyrene resin, NKA9 polystyrene resin, D101 polystyrene resin, and SXD11 polystyrene resin. Whether it is a pure polystyrene resin or a modified polystyrene resin, the above amination method may be used for amino modification.

In some embodiments of the present application, the above amination method includes: the polystyrene-type resin and the enamine salt are dispersed in the solvent, to form a system to be reacted; and in a nitrogen or inert atmosphere, the system to be reacted is mixed with the catalyst and heated to the temperature of the Friedel-Crafts alkylation reaction to perform the Friedel-Crafts alkylation reaction, obtaining the aminated polystyrene-type resin. The enamine salt and the polystyrene-type resin are dispersed in the solvent, so that the enamine salt may enter the pores of the polystyrene-type resin in advance, then the catalyst is added for the Friedel-Crafts alkylation reaction, ensuring faster reaction and a uniform alkylation.

In another typical embodiment of the present application, a method for immobilizing an enzyme using an aminated polystyrene-type resin is provided, and the method includes: the aminated polystyrene-type resin is prepared by any one of the above amination methods; and the aminated polystyrene-type resin is used as a carrier, to immobilize the enzyme by using glutaraldehyde as a crosslinking agent.

The aforementioned preparation method for aminoated polystyrene-type resins benefits from merits such as facile operation and low production cost. The degree of amination can be flexibly adjusted in this aminoation method, thereby controlling the degree of enzyme immobilization in the next steps.

In some embodiments of the present application, the above method includes: the aminated polystyrene-type resin is modified with a buffered solution of glutaraldehyde, to obtain a glutaraldehyde-modified resin; and enzyme solution is crosslinked with the glutaraldehyde-modified resin to achieve immobilization.

In the above method firstly uses the glutaraldehyde to modify the resin, as to use the amino group on the polystyrene-type resin to crosslink with the glutaraldehyde to form a crosslinking network; and then, amine groups on the enzyme is crosslinked with the glutaraldehyde to achieve crosslinking and curing of the enzyme. This procedure makes the crosslinking and curing efficiency of the enzyme higher, especially for enzymes that are sensitive to the glutaraldehyde.

In other embodiments of the present application, the above method includes: the enzyme solution is mixed with the aminated polystyrene-type resin, to obtain a resin adsorbed with the enzyme; and the resin adsorbed with the enzyme is crosslinked with a buffered solution of glutaraldehyde, to achieve immobilization.

In the above method, the enzymes are firstly adsorbed onto the internal and external surfaces of the polystyrene-type resin by the adsorption action, and then reacts with the glutaraldehyde, to achieve the use of the glutaraldehyde to immobilize the enzyme on the polystyrene-type resin by a crosslinking mode. The above method is particularly applicable to enzymes that are not sensitive to the glutaraldehyde.

The buffered solution for glutaraldehyde used in the present application may be a conventional buffer solution used for crosslinking and curing. For example, the above buffer solution of the glutaraldehyde includes the phosphate buffer solution. In addition, in order to improve the immobilization rate of enzyme, preferably the mass content of the glutaraldehyde in the above buffered solution of the glutaraldehyde is 1~2%, as to improve the utilization rate of the glutaraldehyde. In the process of immobilizing the enzyme, the loading amount may be adjusted by varying the ratio of enzyme to the carrier, which is already well known in the art.

Since the above immobilization uses the crosslinking action between the aldehyde group of the glutaraldehyde and the amino group on the enzyme, the method of the present application is applicable to conventional enzymes, for example, the above enzyme is selected from a transaminase.

The beneficial effects of the present application are further described below in combination with embodiments and contrast examples.

### EXAMPLES of amination

### Example 1-1

### Synthesis of polystyrene resin

150 mL of aqueous solution containing 0.5% polyvinyl alcohol (PVA) was added to a 500 mL four-necked flask, and the temperature was increased to 40°C. 16 g of styrene, 5 g of divinylbenzene (80%), 10 mL of toluene, 20 mL of n-heptane, and 100 mg of benzoyl peroxide were mixed evenly, and then added into the four-necked flask. The droplet size were adjusted to an appropriate range by controlling stirring speed, and the temperature was increased to 80°C and kept for 2 h, and then 88°C and kept for 4 h. After the reaction was completed, it was cooled to room temperature, it was filtered to obtain a target bead body. The obtained polystyrene resins were washed sequentially with hot water, ethanol, and deionized water, and finally screened and dried.

### Amination of polystyrene resin

1 g of a dried polystyrene resin and 1 g of 3-butenamine hydrochloride were dispersed into 100 mL of 1,2-dichloroethane and stirred at room temperature for 1 h, to form a system to be reacted. Then 2 g of anhydrous aluminum trichloride was weighed and added to the above mixture and the stirring was allowed to continue for another 10 min. Then the temperature was increased to 80 °C and the reaction was allowed to go on for another 20 h under the protection of N₂. After the reaction was completed, it was cooled to room temperature and washed sequentially with methanol and deionized water to obtain an aminated polystyrene resin: PS-NH₂.

### Example 1-2

### Synthesis of polystyrene-methacrylate resin

240 mL of aqueous solution containing 0.5% PVA and 10% NaCl was added to a 500 mL four-necked flask, and the temperature was increased to 40 °C. 6 g of methyl methacrylate, 15 g of divinylbenzene (80%), 20 mL of 1,2-dichloroethane, 40 mL of methylcyclohexane and 100 mg of azobisisobutyronitrile were mixed evenly, and then added into the four-necked flask. The droplet size were adjusted to an appropriate range by controlling stirring speed, and the temperature was increased to 70 °C and kept for 2 h, and then 80 °C and kept for 4 h, and finally 85 °C for 1 h. After the reaction was completed, it was cooled to a room temperature, it was filtered to obtain a target bead body. The obtained PDMA resins were washed sequentially with hot water, ethanol, and deionized water, and finally screened and dried.

### PDMA resin modification

1 g of a dried PDMA resin and 0.7 g of 3-butenamine hydrochloride were dispersed into 100 mL of 1,2-dichloroethane and stirred at room temperature for 1 h, to form a system to be reacted. Then 2 g of anhydrous aluminum trichloride was weighed and added to the above mixture and the stirring was allowed to continue for another 10 min. Then the temperature was increased to 80 °C and the reaction was allowed to go on for another 20 h under the protection of N₂. After the reaction was completed, it was cooled to room temperature and washed sequentially with methanol and deionized water to obtain an aminated polystyrene resin: PDMA-NH₂.

### Example 1-3

1 g of a dried ECR1090 polystyrene resin (purchased from Purlite) and 1 g of 3-butenamine hydrochloride were dispersed into 100 mL of 1,2-dichloroethane and stirred at room temperature for 1 h, to form a system to be reacted. Then 2 g of anhydrous aluminum trichloride was weighed and added to the above mixture and the stirring was allowed to continue for another 10 min. Then the temperature was increased to 80 °C and the reaction was allowed to go on for another 20 h under the protection of N₂. After the reaction was completed, it was cooled to room temperature and washed sequentially with methanol and deionized water to obtain an aminated polystyrene resin: ECR1090-NH₂.

### Example 1-4

1 g of a dried AB8 resin (purchased from Tianjin Nankai Hecheng Science & Technology Co., Ltd.) and 1 g of 3-butenamine hydrochloride were dispersed into 100 mL of 1,2-dichloroethane and stirred at room temperature for 1 h, to form a system to be reacted. Then 2 g of anhydrous aluminum trichloride was weighed and added to the above mixture and the stirring was allowed to continue for another 10 min. Then the temperature was increased to 80 °C and the reaction was allowed to go on for another 20 h under the protection of N₂. After the reaction was completed, it was cooled to room temperature and washed sequentially with methanol and deionized water to obtain an aminated AB8 resin: AB8-NH₂.

### Example 1-5

1 g of a dried NKA9 resin (purchased from Tianjin Nankai Hecheng Science & Technology Co., Ltd.) and 1 g of 3-butenamine hydrochloride were dispersed into 100 mL of 1,2-dichloroethane and stirred at room temperature for 1 h, to form a system to be reacted. Then 2 g of anhydrous aluminum trichloride was weighed and added to the above mixture and the stirring was allowed to continue for another 10 min. Then the temperature was increased to 80 °C and the reaction was allowed to go on for another 20 h under the protection of N₂. After the reaction was completed, it was cooled to room temperature and washed sequentially with methanol and deionized water to obtain an aminated NKA9 resin: NKA9-NH₂.

### Example 1-6

1 g of a dried SXD-11 resin (purchased from Anhui Sanxing Co., Ltd.) and 1 g of 3-butenamine hydrochloride were dispersed into 100 mL of 1,2-dichloroethane and stirred at room temperature for 1 h, to form a system to be reacted. Then 2 g of anhydrous aluminum trichloride was weighed and added to the above mixture and the stirring was allowed to continue for another 10 min. Then the temperature was increased to 80 °C and the reaction was allowed to go on for another 20 h under the protection of N₂. After the reaction was completed, it was cooled to room temperature and washed sequentially with methanol and deionized water to obtain an aminated SXD-11 resin: SXD-11-NH₂.

### Example 1-7

1 g of a dried D101 resin (purchased from Anhui Sanxing Co., Ltd.) and 1 g of 3-butenamine hydrochloride were dispersed into 100 mL of 1,2-dichloroethane and stirred at room temperature for 1 h, to form a system to be reacted. Then 2 g of anhydrous aluminum trichloride was weighed and added to the above mixture and the stirring was allowed to continue for another 10 min. Then the temperature was increased to 80 °C and the reaction was allowed to go on for another 20 h under the protection of N₂. After the reaction was completed, it was cooled to room temperature and washed sequentially with methanol and deionized water to obtain an aminated D101 resin: D101-NH₂.

The amino contents of the aminated polystyrene resins obtained from the above examples are detected by ninhydrin color development method and acid-base titration method, their specific surface areas and pore sizes are measured by nitrogen adsorption-desorption method. The results are shown in Table 1.

**Table 1**

| Name | Specific surface area (m² g⁻¹) | Average pore size (nm) | Amino content (mmol/g) |
|---|---|---|---|
| PS-NH₂ | 750 | 2.3 | 5.2 |
| PDMA-NH₂ | 420 | 10 | 2.1 |
| AB8-NH₂ | 520 | 8 | 3.8 |
| ECR1090-NH₂ | 610 | 50 | 2.8 |
| NKA9-NH₂ | 150 | 10 | 3.2 |
| D101-NH₂ | 450 | 5 | 3.8 |
| SXD11-NH₂ | 400 | 20 | 2.5 |

### Examples of enzyme immobilization

A transaminase is used as a model, and a transaminase derived from *Chromobacterium violaceum* DSM30191 (CVTA) is used as a model substance, and its sequence SEQ ID NO.1 is:

MQKQRTCSQWRELDAAHHLHPFTDTASLNQAGARVMTRGEGVYLWDCEGNKIIDGMAG
LWCVNVGYGRKDFAEAARRQMEELPFYNTFFGTTHPPVVELSSLLAEVTPAGFDRVFYTNSGS
ESVDTMIRMVRRYWDVQGKPEKKTLIGRWNGYHGSTIGGASLGGMKYMHEQGDLPIPGMAHI

EQPWWYKHGKDMTPDEFGVVAARWLEEKILEIGADKVAAFVGEPIQGAGGVIVPPATYWPEIE
RICRKYDVLLVADEVICGFGRTGEWFGHQHFGFQPDLFTAAKGLSSGYLPLGAVFVGDRVAEG
LIAGGDFNHGFTYSGHPVCAAVAHANVAALRDEGIVQRVKDDIGPYMQKRWRETFSRFEHVDD
VRGVGMMLAFTLVKNKAKRELFPDFGEIGTLCEDIFFRNNLIMTAQGDHIVSAPPLVMTRAEVDE
MLAVAERCLEEFEQTLKARGLA.

### Example 2-1

1% glutaraldehyde solution was prepared with 20 mM phosphate buffer solution (pH = 7.0). 1 g of the aminated polystyrene resin and a pristine polystyrene resin in Example 1-1 were weighed respectively and dispersed into the above solution. It was incubated at 30 °C for 1 h, and then washed three times with deionized water, to obtain a red glutaraldehyde-modified aminated polystyrene resin and a glutaraldehyde-modified pristine polystyrene resin. The resin might become red brown after being modified with the glutaraldehyde, so it was judged whether the glutaraldehyde is successfully modified according to the color change.

1 g of glutaraldehyde-modified aminated polystyrene resin and glutaraldehyde-modified pristine polystyrene resin were added to an enzyme solution (4 mL, 1 V enzyme solution plus 3 V phosphate buffer solution) to prepare an aminated polystyrene immobilized enzyme and a pristine polystyrene resin immobilized enzyme.

The enzyme loading amount was calculated by measuring the difference of the protein concentration before and after immobilization. The loading of enzyme on the aminated polystyrene immobilized enzyme was 26.44 mg/g; and the loading of enzyme on the pristine polystyrene resin immobilized enzyme was 11.09 mg/g.

### Example 2-2

1% glutaraldehyde solution was prepared with 20 mM phosphate buffer solution (pH = 7.0). 1 g of the PDMA-NH₂ resin and PDMA resin in Example 1-2 were weighed respectively and dispersed into the above solution. It was incubated at 30 °C for 1 h, and then washed three times with deionized water, to obtain a red glutaraldehyde-modified PDMA resin and a glutaraldehyde-modified PDMA-NH₂ resin.

1 g of glutaraldehyde-modified PDMA resin and glutaraldehyde-modified PDMA-NH₂ resin were added to an enzyme solution (4 mL, 1 V enzyme solution plus 3 V phosphate buffer solution) to prepare PDMA-NH₂ and PDMA immobilized enzymes, respectively.

The loading of enzyme on the PDMA-NH₂ immobilized enzyme was 28.35 mg/g; and the loading of enzyme on the PDMA immobilized enzymes was 9.17 mg/g.

### Example 2-3

1% glutaraldehyde solution was prepared with 20 mM phosphate buffer solution (pH = 7.0). 1 g of the ECR1090-NH₂ resin and ECR1090 resin in Example 1-3 were weighed respectively and dispersed into the above solution. It was incubated at 30 °C for 1 h, and then washed three times with deionized water, to obtain a red glutaraldehyde-modified ECR1090 resin and a glutaraldehyde-modified ECR1090-NH₂ resin.

1 g of glutaraldehyde-modified ECR1090-NH₂ resin and glutaraldehyde-modified ECR1090 resin were added to an enzyme solution (4 mL, 1 V enzyme solution plus 3 V phosphate buffer solution) to prepare ECR1090-NH₂ and ECR1090 immobilized enzymes, respectively.

The loading of enzyme on the ECR1090-NH₂ immobilized enzyme was 21.82 mg/g; and the loading of enzyme on the ECR1090 immobilized enzyme was 21.09 mg/g.

### Example 2-4

1% glutaraldehyde solution was prepared with 20 mM phosphate buffer solution (pH = 7.0). 1 g of the AB8-NH₂ resin and AB8 resin in Example 1-4 were weighed respectively and dispersed into the above solution. It was incubated at 30 °C for 1 h, and then washed three times with deionized water, to obtain a red glutaraldehyde-modified AB8 resin and a glutaraldehyde-modified AB8-NH₂ resin.

1 g of glutaraldehyde-modified AB8-NH₂ resin and glutaraldehyde-modified AB8 resin were added to an enzyme solution (4 mL, 1 V enzyme solution plus 3 V phosphate buffer solution) to prepare AB8-NH₂ and AB8 immobilized enzymes, respectively.

The loading of enzyme on the AB8-NH₂ immobilized enzyme was 28.37 mg/g; and the loading of enzyme on the AB8 immobilized enzyme was 12.91 mg/g.

### Example 2-5

1% glutaraldehyde solution was prepared with 20 mM phosphate buffer solution (pH = 7.0). 1 g of the NKA9-NH₂ resin and NKA9 resin in Example 1-5 were weighed respectively and dispersed into the above solution. It was incubated at 30 °C for 1 h, and then washed three times with deionized water, to obtain a red glutaraldehyde-modified NKA9 resin and a glutaraldehyde-modified NKA9-NH₂ resin.

1 g of glutaraldehyde-modified NKA9-NH₂ resin and glutaraldehyde-modified NKA9 resin were added to an enzyme solution (4 mL, 1 V enzyme solution plus 3 V phosphate buffer solution) to prepare NKA9-NH₂ and NKA9 immobilized enzymes, respectively.

The loading of enzyme on the NKA9-NH₂ immobilized enzyme was 44.56 mg/g; and the loading of enzyme on the NKA9 immobilized enzyme was 42.31 mg/g.

### Example 2-6

1% glutaraldehyde solution was prepared with 20 mM phosphate buffer solution (pH = 7.0). 1 g of the SXD-11-NH₂ resin and SXD-11 resin in Example 1-5 were weighed respectively and dispersed into the above solution. It was incubated at 30 °C for 1 h, and then washed three times with deionized water, to obtain a red glutaraldehyde-modified SXD-11 resin and a glutaraldehyde-modified SXD-11-NH₂ resin.

1 g of glutaraldehyde-modified SXD-11-NH₂ resin and glutaraldehyde-modified SXD-11 resin were added to an enzyme solution (4 mL, 1 V enzyme solution plus 3 V phosphate buffer solution) to prepare SXD-11-NH₂ and SXD-11 immobilized enzymes, respectively.

The loading of enzyme on the SXD-11-NH₂ immobilized enzyme was 13.31 mg/g; and the loading of enzyme on the SXD-11 immobilized enzyme was 13.41 mg/g.

The following enzymatic reaction was employed:

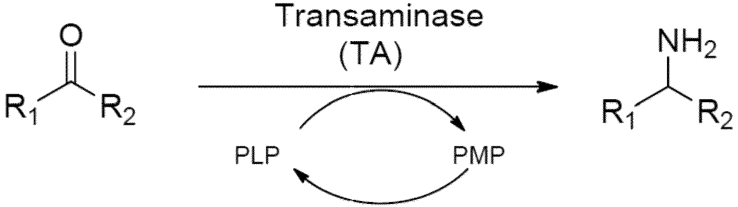

In a 20 mL reaction flask, 0.5 mL of methanol was added to dissolve 0.1 g of a carbonyl substrate, and 15 eq of isopropylamine hydrochloride and 25.0 mg of 5'-pyridoxal phosphate (PLP) were added, 0.1 M phosphate buffer solution (pH 8.0) was added until the final volume of reaction solution reached 5 mL, to form a system to be reacted; and

0.1 g of transaminase powder or 0.1 g of an immobilized enzyme (prepared with the transaminase powder) was respectively added to the system to be reacted independently, and the mixture was stirred at 47 °C for 20 h. The conversion rate of the system was detected by high performance liquid chromatography (HPLC). After each round of the reaction, the immobilized enzymes were separated and used in the next round of the reaction to test their reusability. The results were shown in Table 2.

**Table 2**

| Enzyme | Example | Carrier | Conversion rate (%) | Cycle count |
|---|---|---|---|---|
| | | Free enzyme | 99 | 1 |
| | Example 2-1 | PS | 9 | 1 |
| | | PS-NH₂ | 87 | 10 |
| | Example 2-2 | PDMA | 13 | 1 |
| | | PDMA-NH₂ | 90 | 10 |
| Transaminase derived from *Chromobacterium violaceum* DSM30191 | Example 2-3 | ECR1090 | 20 | 1 |
| | | ECR1090-NH₂ | 98 | 10 |
| | Example 2-4 | AB8 | 1 | 1 |
| | | AB8-NH₂ | 80 | 10 |
| | Example 2-5 | NK9 | 36 | 1 |
| | | NK9-NH₂ | 95 | 10 |
| | Example 2-6 | SXD-11 | 3 | 1 |
| | | SXD-11-NH₂ | 95 | 10 |

From the above description, it can be seen that the above examples of the present disclosure achieved the following technical effects.

The present application utilizes the Friedel-Crafts alkylation reaction to graft the enamine salt onto the polystyrene-type resin, and the amination of the polystyrene-type resin is completed. The conditions of the Friedel-Crafts alkylation reaction are easy to control, the post-treatment process is simple, and it is only necessary to remove the catalyst and the unreacted enamine salt by washing. Therefore, the above amination method in the present application has few steps and is simple and easy to implement. Meanwhile, the amination method further avoids the use of a noble metal catalyst, thereby the production cost is reduced. The commercial polystyrene resins modified by aforementioned amination method may be successfully used as an enzyme immobilization carrier, and exhibit good immobilization effects and reusability. The present application also provides new insights for the design of enzyme immobilization carrier, so that more selections for immobilization of the different enzymes are provided in terms of carrier pore size, specific surface area, skeleton structure, carrier polarity, and other aspects.

## Claims

1. An amination method for a polystyrene-type resin, wherein the amination method comprises: in a solvent, catalyzing the polystyrene-type resin and an enamine salt by a catalyst for a Friedel-Crafts alkylation reaction, to obtain an aminated polystyrene-type resin, wherein the catalyst is a Lewis acid catalyst.

2. The amination method according to claim 1, wherein a temperature of the Friedel-Crafts alkylation reaction is 40-80 °C, and a time of the Friedel-Crafts alkylation reaction is preferably 12-20 h.

3. The amination method according to claim 1, wherein a mass ratio of the polystyrene-type resin and the enamine salt is 2:1 to 2:3.

4. The amination method according to claim 1, wherein a molar ratio of the enamine salt and the Lewis acid catalyst is 1:3 to 1:5.

5. The amination method according to claim 1, wherein the Lewis acid catalyst is any one of anhydrous aluminum trichloride and iron trichloride or a combination thereof.

6. The amination method according to claim 1, wherein the enamine salt is 3-butenylamine hydrochloride.

7. The amination method according to claim 1, wherein the solvent is 1,2-dichloroethane.

8. The amination method according to claim 1, wherein the polystyrene-type resin is selected from any one of a polystyrene resin or a polystyrene-methacrylate resin.

9. The amination method according to any one of claims 1 to 6, wherein the amination method comprises:
dispersing the polystyrene-type resin and the enamine salt in the solvent, to form a system to be reacted; and
in a nitrogen or inert atmosphere, after mixing the system to be reacted with the catalyst, heating to the temperature of Friedel-Crafts alkylation reaction and performing the Friedel-Crafts alkylation reaction, to obtain the aminated polystyrene-type resin.

10. A method for immobilizing an enzyme with an aminated polystyrene-type resin, wherein the method comprises:
preparing the aminated polystyrene-type resin by the amination method according to any one of claims 1 to 9; and
immobilizing the enzyme with the aminated polystyrene-type resin as a carrier, and the glutaraldehyde as a cross-linking agent.

11. The method according to claim 10, wherein the method comprises:
modifying the aminated polystyrene-type resin by a buffer solution of the glutaraldehyde, to obtain a glutaraldehyde-modified resin; and
performing a cross-linking reaction on a enzyme solution and the glutaraldehyde-modified resin, to achieve the immobilization.

12. The method according to claim 10, wherein the method comprises:
mixing the enzyme solution with the aminated polystyrene-type resin, to obtain an enzyme-adsorbed resin; and
performing a cross-linking reaction on the enzyme-adsorbed resin and a buffer solution of the glutaraldehyde, to achieve the immobilization.

13. The method according to claim 11 or 12, wherein the buffer solution of the glutaraldehyde comprises a phosphate buffer solution, a mass content of the glutaraldehyde in the buffer solution of the glutaraldehyde is 1-2%, and the enzyme is a transaminase.

## Patentansprüche

1. Aminierungsverfahren für ein polystyrolartiges Harz, wobei das Aminierungsverfahren umfasst:
in einem Lösungsmittel, Katalysieren des polystyrolartigen Harzes und eines Enaminsalzes durch einen Katalysator für eine Friedel-Crafts-Alkylierungsreaktion, um ein aminiertes polystyrolartiges Harz zu erhalten, wobei der Katalysator ein Lewis-Säure-Katalysator ist.

2. Aminierungsverfahren nach Anspruch 1, wobei eine Temperatur der Friedel-Crafts-Alkylierungsreaktion 40-80 °C beträgt und eine Zeit der Friedel-Crafts-Alkylierungsreaktion vorzugsweise 12-20 h beträgt.

3. Aminierungsverfahren nach Anspruch 1, wobei ein Massenverhältnis zwischen dem polystyrolartigen Harz und dem Enaminsalz 2:1 bis 2:3 beträgt.

4. Aminierungsverfahren nach Anspruch 1, wobei ein Molverhältnis zwischen dem Enaminsalz und dem Lewis-Säure-Katalysator 1:3 bis 1:5 beträgt.

5. Aminierungsverfahren nach Anspruch 1, wobei der Lewis-Säure-Katalysator entweder wasserfreies Aluminiumtrichlorid oder Eisentrichlorid oder eine Kombination davon ist.

6. Aminierungsverfahren nach Anspruch 1, wobei das Enaminsalz 3-Butenylaminhydrochlorid ist.

7. Aminierungsverfahren nach Anspruch 1, wobei das Lösungsmittel 1,2-Dichlorethan ist.

8. Aminierungsverfahren nach Anspruch 1, wobei das polystyrolartige Harz entweder aus einem Polystyrolharz oder einem Polystyrolmethacrylatharz ausgewählt ist.

9. Aminierungsverfahren nach einem der Ansprüche 1 bis 6, wobei das Aminierungsverfahren umfasst:
Dispergieren des polystyrolartigen Harzes und des Enaminsalzes in dem Lösungsmittel, um ein System zu bilden, das zur Reaktion gebracht werden soll; und
in einer Stickstoff- oder inerten Atmosphäre nach Vermischen des zur Reaktion zu bringenden Systems mit dem Katalysator, Erhitzen auf die Temperatur der Friedel-Crafts-Alkylierungsreaktion und Durchführen der Friedel-Crafts-Alkylierungsreaktion, um das aminierte polystyrolartige Harz zu erhalten.

10. Verfahren zur Immobilisierung eines Enzyms mit einem aminierten polystyrolartigen Harz, wobei das Verfahren umfasst:
Herstellen des aminierten polystyrolartigen Harzes durch das Aminierungsverfahren nach einem der Ansprüche 1 bis 9; und
Immobilisieren des Enzyms mit dem aminierten polystyrolartigen Harz als Träger und dem Glutaraldehyd als Vernetzungsmittel.

11. Verfahren nach Anspruch 10, wobei das Verfahren umfasst:
Modifizieren des aminierten polystyrolartigen Harzes durch eine Pufferlösung des Glutaraldehyds, um ein Glutaraldehyd-modifiziertes Harz zu erhalten; und
Durchführen einer Vernetzungsreaktion an einer Enzymlösung und dem Glutaraldehyd-modifizierten Harz, um die Immobilisierung zu erreichen.

12. Verfahren nach Anspruch 10, wobei das Verfahren umfasst:
Mischen der Enzymlösung mit dem aminierten polystyrolartigen Harz, um ein enzymadsorbiertes Harz zu erhalten; und
Durchführen einer Vernetzungsreaktion an dem enzymadsorbierten Harz und einer Pufferlösung des Glutaraldehyds, um die Immobilisierung zu erreichen.

13. Verfahren nach Anspruch 11 oder 12, wobei die Pufferlösung des Glutaraldehyds eine Phosphatpufferlösung umfasst, ein Massengehalt des Glutaraldehyds in der Pufferlösung des Glutaraldehyds 1-2 % beträgt und das Enzym eine Transaminase ist.

## Revendications

1. Procédé d'amination d'une résine de type polystyrène, le procédé d'amination comprenant :
dans un solvant, la catalysation de la résine de type polystyrène et d'un sel d'énamine par un catalyseur pour une réaction d'alkylation de Friedel-Crafts, afin d'obtenir une résine aminée de type polystyrène, le catalyseur étant un catalyseur acide de Lewis.

2. Procédé d'amination selon la revendication 1, la température de la réaction d'alkylation de Friedel-Crafts étant de 40 à 80 °C, et la durée de la réaction d'alkylation de Friedel-Crafts étant de préférence 12-20 heures.

3. Procédé d'amination selon la revendication 1, le rapport massique entre la résine de type polystyrène et le sel d'énamine étant de 2:1 à 2:3.

4. Procédé d'amination selon la revendication 1, le rapport molaire entre le sel d'énamine et le catalyseur acide de Lewis étant de 1:3 à 1:5.

5. Procédé d'amination selon la revendication 1, le catalyseur acide de Lewis étant l'un quelconque des trichlorures d'aluminium et de fer anhydres ou une combinaison de ceux-ci.

6. Procédé d'amination selon la revendication 1, le sel d'énamine étant le chlorhydrate de 3-buténylamine.

7. Procédé d'amination selon la revendication 1, le solvant étant le 1,2-dichloroéthane.

8. Procédé d'amination selon la revendication 1, la résine de type polystyrène étant choisie parmi une résine de polystyrène ou une résine de polystyrène-méthacrylate.

9. Procédé d'amination selon l'une quelconque des revendications 1 à 6, le procédé d'amination comprenant :
la dispersion de la résine de type polystyrène et du sel d'énamine dans le solvant, pour former un système à faire réagir ; et
dans une atmosphère d'azote ou inerte, après avoir mélangé le système à faire réagir avec le catalyseur, le chauffage à la température de la réaction d'alkylation de Friedel-Crafts et l'exécution de la réaction d'alkylation de Friedel-Crafts, pour obtenir la résine aminée de type polystyrène.

10. Procédé d'immobilisation d'une enzyme avec une résine aminée de type polystyrène, le procédé comprenant :
la préparation de la résine aminée de type polystyrène par le procédé d'amination selon l'une quelconque des revendications 1 à 9 ; et
l'immobilisation de l'enzyme avec la résine aminée de type polystyrène comme support et du glutaraldéhyde comme agent de réticulation.

11. Procédé selon la revendication 10, le procédé comprenant :
la modification de la résine aminée de type polystyrène par une solution tampon de glutaraldéhyde, afin d'obtenir une résine modifiée par le glutaraldéhyde ; et
la réalisation d'une réaction de réticulation sur une solution enzymatique et la résine modifiée par le glutaraldéhyde, afin d'accomplir l'immobilisation.

12. Procédé selon la revendication 10, le procédé comprenant :
le mélange de la solution enzymatique avec la résine aminée de type polystyrène, pour obtenir une résine adsorbée par l'enzyme ; et
la réalisation d'une réaction de réticulation sur la résine adsorbée par l'enzyme et une solution tampon de glutaraldéhyde, afin d'accomplir l'immobilisation.

13. Procédé selon la revendication 11 ou 12, la solution tampon de glutaraldéhyde comprenant une solution tampon de phosphate, une teneur en masse de glutaraldéhyde dans la solution tampon de glutaraldéhyde étant de 1-2 %, et l'enzyme étant une transaminase.
